Europäisches Patentamt

European Patent Office

Office européen des brevets

(11) Veröffentlichungsnummer: **0 394 731**
**A1**

## EUROPÄISCHE PATENTANMELDUNG

(21) Anmeldenummer: 90106723.1

(22) Anmeldetag: 07.04.90

(51) Int. Cl.⁵: **C08L 5/00, C08L 1/28,**
**C08K 3/32, C08K 5/09,**
**C08K 5/17, A23L 1/054,**
**A61K 9/00**

(30) Priorität: 24.04.89 DE 3913402

(43) Veröffentlichungstag der Anmeldung:
**31.10.90 Patentblatt 90/44**

(84) Benannte Vertragsstaaten:
**AT BE DE DK FR GB IT LU NL SE**

(71) Anmelder: **Wolff Walsrode Aktiengesellschaft**
**Postfach**
**D-3030 Walsrode 1(DE)**

(72) Erfinder: **Wilke, Michaela, Dr.**
**Ahornweg 9**
**D-3043 Schneverdingen(DE)**
Erfinder: **Szablikowski, Klaus, Dr.**
**Claudiusstrasse 5**
**D-3030 Walsrode(DE)**

(74) Vertreter: **Zobel, Manfred, Dr. et al**
**c/o BAYER AG Konzernverwaltung RP**
**Patente Konzern**
**D-5090 Leverkusen 1 Bayerwerk(DE)**

(54) Verfahren zur Herstellung von Mischungen aus Xanthan und Cellulosederivaten.

(57) Mischungen aus cellulasefreiem Xanthan und wenigstens einem Cellulosederivat mit verbesserter Löslichkeit werden dadurch hergestellt, daß das Xanthan und das Cellulosederivat in einem Wirbelbett unter Aufsprühen eines Hilfsstoffes vermischt werden.

## Verfahren zur Herstellung von Mischungen aus Xanthan und Cellulosederivaten

Die unterschiedlichsten Mischungen von Verdickern (Gums) sind bekannt. Mischungen von Xanthan mit Cellulose und/oder Cellulosederivaten waren davon bisher weitestgehend ausgenommen, da Xanthan in der Regel Cellulasen enthält. Das mit cellulytisch aktiven Enzymen behaftete trockene Xanthan hat daher den Nachteil, in Lösung nicht mit Cellulose oder Cellulosederivaten kombinierbar zu sein. Bei Kombination eines cellulasenhaltigen Xanthans mit z.B. Carboxymethylcellulose in Lösung kommt es nach relativ kurzer Zeit zu einer Viskositätserniedrigung.

In EP-A-00 48 616 werden zwar Mischungen von cellulasefreiem Xanthan mit einigen Cellulosederivaten beschrieben, die für die Lösung der Mischung in wäßrigen Systemen erforderliche Zeit ist aber zu lang.

Der Erfindung lag die Aufgabe zugrunde, Mischungen aus cellulasefreiem Xanthan und Cellulosederivaten mit verbesserter Löslichkeit und verbessertem rheologischem Verhalten bereitzustellen.

Gegenstand der Erfindung ist ein Verfahren zur Herstellung von Mischungen mit verbesserter Löslichkeit aus cellulasefreiem Xanthan und wenigstens einem Cellulosederivat, dadurch gekennzeichnet, daß das Xanthan und das Cellulosederivat in einem geeigneten Mischaggregat unter Aufsprühen von Hilfsstoffen vermischt werden.

Bevorzugte Cellulosederivate sind Celluloseether, insbesondere Carboxymethylcellulose, Methylcellulose, Hydroxypropylmethylcellulose und Sulfoethylcellulose.

Es können übliche bekannte cellulasefreie Xanthane eingesetzt werden, besonders bevorzugt ist aber ein Xanthan, welches gemäß der Deutschen Patentanmeldung 38 12 682 dadurch gereinigt worden ist, daß man

a) eine cellulasehaltiges Xanthan enthaltene Lösung auf einen pH-Wert außerhalb des Bereiches von 5 bis 9, einstellt

b) anschließend eine Temperaturbehandlung bei erhöhter Temperatur durchführt

c) gegebenenfalls abkühlt

d) gegebenenfalls neutralisiert und gegebenenfalls

e) das so erhaltene Produkt in ein hochturbulentes Fällbad zur Ausfällung des Xanthans einträgt.

Insbesondere wenn ein klarlösliches Produkt erhalten werden soll, wird in einem Schritt f) vorzugsweise vor dem Schritt a) oder nach dem Schritt d) die das Xanthan enthaltende Lösung einer Filtration unterworfen.

In einer besonders bevorzugten Ausführungsform wird das Xanthan in Stufe a) mit einer Säure auf einen pH-Wert von 1 bis 3, insbesondere 1 bis 2 eingestellt. Bevorzugte Säuren sind anorganische Säuren wie Salzsäure und Salpetersäure sowie organische Säuren wie Brenztraubensäure und Essigsäure.

In einer besonders bevorzugten Ausführungsform wird in Stufe b) das Produkt bei einer Temperatur von 40 bis 120 °C, insbesondere 60 bis 100 °C, erhitzt, vorzugsweise 10 bis 120 Minuten, insbesondere 45 bis 90 Minuten.

Das Fällbad in Stufe e) besteht vorzugsweise aus einem Alkohol, insbesondere Isopropanol.

Bei dem aufgesprühten Hilfsstoff handelt es sich vorzugsweise um Lösungen von Substanzen aus der Klasse der Komplexbildner, z.B. Ethylendiamintetraessigsäure (EDTA), der Phosphate, z.B. Kaliumdihydrogenphosphat, oder auch der Carbonsäuren, z.B. Weinsäure, insbesondere aber um Wasser. Bevorzugte Lösungsmittel sind Wasser und Alkohol.

Bei der Verwendung von Carbonsäuren hat es sich als besonders vorteilhaft erwiesen, wenn als zweite Komponente ein Carbonat bzw. Hydrogencarbonat zugesetzt wird. Die Auftragung der beiden Komponenten hat in zwei getrennten Arbeitsgängen zu erfolgen. Die Lösungsmittel bzw. der Hilfsstoff Wasser werden im nachfolgenden Trocknungsschritt wieder verdampft.

Die Menge des im Produkt verbleibenden, nicht verdampfenden Hilfsstoffes, bezogen auf die Polymere, liegt bei 0 - 20 Gew.-%, bevorzugt bei 0 - 10 Gew.-%.

Als besonders geeignetes Aggregat hat sich ein sogenannter Wirbelbett-Trockner erwiesen. Die Vermischung der beiden eingesetzten Komponenten erfolgt derart, daß die Trockensubstanzen in den Wirbelbett-Trockner gefüllt werden, ein Wirbelbett durch Einblasen angewärmter Luft aufgebaut und die Hilfsstoffe auf die im Wirbelbett befindlichen Substanzen gesprüht werden. Nach einer gewissen Zeit haben sich aus den eingesetzten pulverigen Substanzen agglomerierte Teilchen gebildet, die beim Auflösen in Wasser das geschilderte besondere rheologische Verhalten zeigen. Andere geeignete Mischaggregate sind beheizbare Mischer mit mechanischen oder pneumatischen Rühr- oder Knetvorrichtungen, wie sie z.B. in Ullmann's Encyclopedia of Technical Chemistry, Bd. B 2, Kapitel 26 und 27, Weinheim, 1988, beschrieben werden.

Für die Mischung im Wirbelbett-Trockner ist es sinnvoll, daß die eingesetzten Trockenpulver annähernd gleiches Schüttgewicht besitzen, damit es während der Agglomeration nicht zu einer Art Windsichtung und damit einer Abänderung des Mischungsverhältnisses kommt.

Xanthan und Cellulosederivate werden in einem Mischungsverhältnis von 1:4 bis 4:1, vorzugsweise in einem Mischungsverhältnis von 1:2 bis 2:1 eingesetzt.

Wird eine Carbonsäure eingesetzt, so kann entweder nur die Säure als alleiniger Zusatzstoff auf die Mischung aus Xanthan und Cellulosederivat beaufschlagt werden, es ist aber auch die Kombination mit einer zweiten Komponente, bevorzugt einem Carbonat, besonders bevorzugt ein Hydrogencarbonat, möglich. In diesem Fall wird zunächst eine Lösung des Carbonats auf das im Wirbelbett befindliche Gemisch aus Xanthan und Cellulosederivat aufgesprüht. Anschließend wird in einem zweiten Arbeitsgang die Carbonsäure aufgetragen, wobei die Auftragung nicht zwingend im Mischaggregat erfolgen muß, sondern z.B. auch durch Trocknen einer Aufschlämmung, bestehend aus vorbehandeltem Xanthan/Cellulosederivatgemisch und einer alkoholischen Carbonsäurelösung, in einem Rotationsverdampfer erfolgen kann.

Die erfindungsgemäß hergestellten Mischungen zeigen überraschenderweise eine deutlich verbesserte Löslichkeit und darüber hinaus überraschende rheologische Eigenschaften.

Die erfindungsgemäß hergestellten Mischungen lösen sich schneller als bekannte Xanthan/Cellulosederivat-Mischungen. Bevorzugte Mischungen haben einen Lösezeitquotientien LQ von kleiner 1, insbesondere kleiner 0,7. Hierin bedeuten:

$$LQ = \frac{L \; Compound}{L \; Trocken}$$

$L_{Trocken}$ = Lösezeit in Minuten bei 20°C einer Trockenmischung aus Xanthan und einem Cellulosederivat unter folgenden Bedingungen:

a) Zusammensetzung der Mischung: gleiche Gewichtsteile Xanthan und Cellulosederivat

b) 1 g Mischung auf 100 ml Wasser

c) die Lösezeit ist definiert als die Zeit, die bis zum Erreichen der Endviskosität erforderlich ist. $L_{Compound}$ ist die Lösezeit einer erfindungsgemäßen Mischung. Sie wird wie unter a) - c) angegeben bestimmt mit folgenden Änderungen:

a) In der Mischung sind zusätzlich 0 - 20 Gew.-% des Hilfsstoffs vorhanden.

Das rheologische Verhalten wurde sowohl von den Einzelkomponenten als auch von daraus unterschiedlich hergestellten Mischungen untersucht.

Die Analyse erfolgte jeweils durch Aufnahme von sogenannten Fließkurven mit einem Rotationsviskosimeter. Unter Fließkurven versteht man die grafische Darstellung der in einem rheologischen Experiment gewonnenen Daten, indem die Schubspannung $\tau$ gegen das Schergefälle $\gamma$ aufgetragen wird. Zu jeweils einem $\tau/\gamma$-Wertepaar läßt sich die dazugehörige Viskosität $\eta$ rechnerisch bestimmen.

Zur Aufnahme der Fließkurven wurde von einem minimalen Schergefälle in Richtung eines festgelegten maximalen Schergefälles und in umgekehrter Richtung gemessen.

Vergleicht man beide Messungen, eingetragen in ein $\tau/\gamma$-Diagramm, miteinander, so stellt man fest, daß die ermittelten Kurven nicht immer deckungsgleich sind. Liegt die Kurve in Richtung zum maximalen Schergefälle über der Kurve in Richtung des minimalen Schergefälles, so wird diese Erscheinungsform als Thixotropie bezeichnet. Den umgekehrten Fall nennt man Rheopexie. Erfahrungsgemäß ist der Effekt der Rheopexie selten zu beobachten.

Um so überraschender war es daher, daß Mischungen aus Celluloseethern z.B. Carboxymethylcellulose, Hydroxypropylcellulose und Methylcellulose und Xanthan thixotrop oder rheopex sein können. Ob Thixotropie oder Rheopexie vorliegt, ist von verschiedenen Parametern abhängig. Werden Lösungen der Einzelkomponenten miteinander gemischt, so spielt das Mischungsverhältnis der Komponenten Celluloseether und Xanthan eine Rolle.

Beträgt das Mischungsverhältnis z.B. 50 Gew.-% an der Einzelkomponente Celluloseether und 50 Gew.-% an der Einzelkomponente Xanthan, so ist entweder Thixotropie oder Rheopexie feststellbar. Ändert man das Mischungsverhältnis, indem z.B. 33 Gew.-% der einen Einzelkomponente und 66 Gew.-% der anderen Einzelkomponente eingesetzt werden, so ist der jeweilig entgegengesetzte rheologische Effekt feststellbar.

Ist nun das rheologische Verhalten einer Mischung aus Celluloseether und Xanthan bekannt, so kann durch Änderung des Mischungsverhältnisses ein bestimmtes rheologisches Verhalten eingestellt werden.

Grundvoraus setzung ist dafür, daß die Gesamtkonzentration an Einzelkomponenten nicht geändert wird. Die Summe aus den Konzentrationen der Einzelkomponenten beträgt 0,25 bis 1,5 Gew.-%.

Überraschenderweise wurde nun gefunden, daß das rheologische Verhalten auch von der Herstellmethode der Mischungen abhängig ist.

Werden nämlich die Einzelkomponenten, Xanthan und z.B. ein Celluloseether, als Trockensubstanzen nach der Erfindung zugrundeliegenden Methode gemischt, und stellt man aus dieser Mischung wäßrige Lösungen definierter Konzentration her, so findet man ein deutlich geändertes rheologisches Verhalten im Vergleich zu Mischungen der jeweiligen Lösungen von Einzelkomponenten und den aus einem Gemisch hergestellten Lösungen.

Zu einem zeigt sich eine deutlich stärkere Rheopexie. Weiterhin ist feststellbar, daß das rheopexe Verhalten bei allen Konzentrationen der hergestellten Lösungen zu beobachten ist und mit der Konzentration zunimmt. Der bevorzugte Konzentrationsbereich beträgt hierbei 0,25 bis 1,5 Gew.-%.

Bevorzugte erfindungsgemäße Mischungen aus Xanthan und einem Cellulosederivat weisen eine Hysteresefläche H mit negativem Vorzeichen, d.h. Rheopexie, auf. Der Absolutwert der Hysteresefläche H von erfindungsgemäßen Mischungen ist hoher als der von Trockenmischungen oder Lösungsgemischen. Die Hysteresefläche wird wie folgt bestimmt:

1. Verwendet wird eine erfindungsgemäße Mischung aus einem Xanthan und einem Cellulosederivat in Gewichtsverhältnis 1:1.

2. Hiervon wird eine 1 gew.-%ige Lösung in Wasser hergestellt.

3. Bei 20° C wird an dieser Lösung die Schubspannung in Abhängigkeit von der Schergeschwindigkeit mittels eines Rotationsviskosimeters mit Couette-System nach DIN 53 018 kontinuierlich innerhalb von 3 Minuten im Schergeschwindigkeitsberich von $\gamma$ = 0 bis 50 $s^{-1}$ (Aufwärtskurve) und unmittelbar anschließend innerhalb von 3 Minuten von 50 bis 0 $s^{-1}$ (Abwärtskurve) gemessen.

4. Die Hysteresefläche H ergibt sich aus der Flächendifferenz - Fläche unter der Aufwärtskurve - Fläche unter der Abwärtskurve in dem Schergeschwindigkeitsbereich von $\gamma$ = 0 bis 50 $s^{-1}$.

Verwiesen wird in diesem Zusammenhang auf die entsprechenden Darstellungen in W.M. Kulicke, Fließverhalten von Stoffen und Stoffgemischen, 1. Auflage, Basel 1986, Hüthig u. Wepf, insbesondere Seiten 36 ff.

Mögliche Anwendungsgebiete für die erfindungsgemäßen Mischungen aus Cellulosederivaten und cellulasenfreiem Xanthan sind in den Bereichen Lebensmittel, Kosmetik, Pharma und technische Anwendungen zu finden.

Beispiele

Beispiel 1

In einem Wirbelbett-Trockner der Fa. Büchi, Modell 710, werden 25 g cellulasenfreies, pulveriges Trocken-Xanthan und 25 g pulverige, trockene Carboxymethylcellulose gegeben. Zur Erzeugung des Wirbelbetts werden folgende Geräteparameter eingestellt: Aspirator 0-1 SKT, Filterreinigung 3-10 SKT, Spraydruck 0,8-1,0 bar, Pumpe 4,5 SKT, Produkt-Temperatur ca. 60° C. Nachdem sich das Wirbelbett eingestellt hatte, wurden ca. 10 bis 30 ml $H_2O$ während 20 Minuten auf die Trockensubstanzen bis zur vollständigen Agglomeration über eine Düseneinrichtung gesprüht.

Diese Konfektionierungsform wird im folgenden Compound genannt.

Anschließend wurde diese Mischung in einem Rotationsviskosimeter (Hersteller: Haake, Karlsruhe; Gerätetyp: RV 20; CV 100; ZA 30) bei einer Meßtemperatur von 20° C als 1 %ige wäßrige Lösung vermessen. Es wurde die Fließkurve aufgenommen, wobei $\gamma$ max. = 50 $s^{-1}$.

Als Grundlage zur Beurteilung, ob Thixotropie oder Rheopexie vorliegt, wird die Fläche H zwischen den Kurven im $\tau/\gamma$-Diagramm herangezogen.

Die Ergebnisse sind in Tabelle 1 aufgeführt.

Beispiel 2 (Vergleichsbeispiel)

Carboxymethylcellulose in Pulverform wurde mit trockenem, pulverförmigen Xanthan zu gleichen Teilen vermischt. Die Konfektionierungsform wurde im folgenden Trockenmischung genannt. Aus dieser Trockenmischung wurde eine wäßrige Lösung mit einer Gesamtkonzentration von 1 Gew.-% hergestellt. Die Ergebnisse der rheologischen Untersuchungen sind in Tabelle 1 aufgeführt.


Beispiel 3 (Vergleichsbeispiel)

Es wurden jeweils 1 gew.-%ige wäßrige Lösungen von Carboxymethlcellulose und cellulosefreiem Xanthan zu gleichen Teilen vermischt. Diese Konfektionierungsform wird im folgenden Lösungsgemisch genannt.

Die Ergebnisse der rheologischen Untersuchungen sind in Tabelle 1 aufgeführt.


Beispiel 4 und 5: reine Substanzen.


Beispiele 6 - 15

Mischungen von Xanthan mit Hydroxyethylcellulose bzw. Hydroxypropylcellulose wurden in gleicher Weise, wie in Beispiel 1 - 3 beschrieben, hergestellt. Beispiele 9, 10, 14 und 15 betreffen Einzelsubstanzen, die zum Vergleich vermessen werden. Die Ergebnisse der rheologischen Untersuchungen sind in Tabelle 1 aufgeführt.

Es zeigt sich bei der erfindungsgemäßen Konfektionierung gegenüber der Trockenmischung eine erheblich verringerte Lösungszeit.

Tabelle 1

| Nr. | Konfektionierung | Substanz | Verhältnis | H* (20h) (mPa.s) | Lösezeit (min) | Viskosität nach 20 h Aufwärtskurve (D = 40/s) (mPa.s) | Viskosität nach 20 h Abwärtskurve (D = 40/s) (mPa.s) | Schüttgewicht (g/l) |
|---|---|---|---|---|---|---|---|---|
| 1 | Compound | CMC + XN | 1:1 | -25000 | 36 | 246 | 251 | 102 |
| 2 | Trockenmischung | CMC + XN | 1:1 | -21000 | 77 | 163 | 168 | 243 |
| 3 | Lösungsgemisch | CMC + XN | 1:1 | -2000 | | 135 | 135 | |
| 4 | | Xanthan N | | -9000 | > = 180 | 215 | 217 | 322 |
| 5 | | CMC | | 35000 | | 144 | 145 | 108 |
| 6 | Compound | HPMC + XN | 1:1 | -15000 | 56 | 430 | 432 | 151 |
| 7 | Trockenmischung | HPMC + XN | 1:1 | 0 | > = 180 | 333 | 333 | 406 |
| 8 | Lösungsgemisch | HPMC + XN | 1:1 | -3000 | | 308 | 208 | |
| 9 | | Xanthan N 1.12.88 | | 9000 | > = 180 | 215 | 217 | 322 |
| 10 | | HPMC | | -14000 | | 253 | 255 | 316 |
| 11 | Compound | HEC + XN | 1:1 | -11000 | 103 | 417 | 419 | 177 |
| 12 | Trockenmischung | HEC + XN | 1:1 | -5000 | > = 180 | 496 | 495 | 473 |
| 13 | Lösungsgemisch | HEC + XN | 1:1 | -3000 | | 401 | 399 | |
| 14 | | Xanthan N 1.12.88 | | 9000 | > = 180 | 215 | 217 | 322 |
| 15 | | HEC | | -15000 | | 330 | 331 | 514 |

HPMC: Hydroxypropylmethylcellulose

HEC: Hydroxyethylcellulose

CMC: Carboxymethylcellulose

H: siehe Erläuterung im Beispiel 1

EP 0 394 731 A1

Beispiel 16

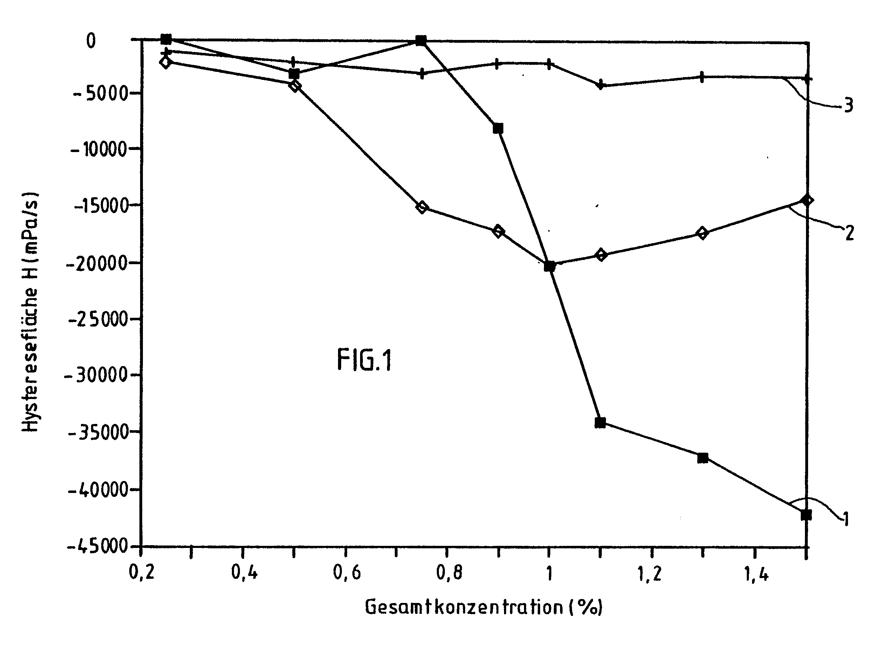

Die in Beispiel 1 - 3 beschriebenen Carboxymethylcellulose-Xanthan-Mischungen wurden in unterschiedlichen Konzentrationen in Wasser gelöst und die rheologischen Eigenschaften bestimmt. Die Ergebnisse sind in der Fig. 1 wiedergegeben.
Hierin bedeuten:

Kurve 1 erfindungsgemäße Mischung
Kurve 2 Trockenmischung der Substanzen, ohne Hilfsstoff
Kurve 3 Mischungen der Substanzen in Lösung.
Es ist deutlich zu sehen, daß erfindungsgemäß ein stark rheopexes Verhalten erreicht werden kann.

Beispiele 17 - 20

12,5 g pulveriges, cellulasefreies Trockenxanthan und 12,5 g pulverige, trockene Carboxymethylcellulose wurden, wie in Beispiel 1 beschrieben, behandelt. Als Sprühlösung wurden in separaten Versuchen je 100 ml einer 2,5 gew.-%igen Lösung folgender Substanzen bzw 30 ml reines Wasser eingesetzt:

17 - Na-EDTA-Lösung
18 - Pentanatriumtriphosphatlösung
19 - Kaliumhydrogenphosphat
20 - reines Wasser
Die Ergebnisse sind in Tabelle 2 aufgeführt.

Beispiel 21

12,5 g pulveriges, cellulasefreies Trockenxanthan und 12,5 g pulverige, trockene Carboxymethylcellulose wurden, wie in Beispiel 1 beschrieben, behandelt. Als Sprühlösung wurden 100 ml einer 2,5 gew.-%gen wäßrigen Natriumhydrogencarbonatlösung verwendet.
Das erhaltene Trockenprodukt wurde in 250 ml einer 2,1 gew.-%igen ethanolischen Weinsäurelösung aufgeschlämmt und am Rotationsverdampfer zur Trockene eingeengt.
Die Ergebnisse sind in der Tabelle 2 aufgeführt.

Tabelle 2

| Beispiel | Lösezeit (min.) | Schüttgewicht (g/l) |
|----------|-----------------|---------------------|
| 17 | 30 | 160 |
| 18 | 25 | 96 |
| 19 | 22 | 165 |
| 20 | 36 | 102 |
| 21 | 18 | 120 |

**Ansprüche**

1. Verfahren zur Herstellung von Mischungen mit verbesserter Löslichkeit aus cellulasefreiem Xanthan und wenigstens einem Cellulosederivat, dadurch gekennzeichnet, daß das Xanthan und das Cellulosederivat in einem geeigneten Mischaggregat unter Aufsprühen eines Hilfsstoffes vermischt werden.
2. Verfahren nach Anspruch 1, dadurch gekennzeichnet, daß das Cellulosederivat ein Celluloseether ist.
3. Verfahren nach Anspruch 1, dadurch gekennzeichnet, daß das Cellulosederivat Carboxymethylcellulose, Methylcellulose, Hydroxypropylmethylcellulose oder Sulfoethylcellulose ist.

4. Verfahren nach wenigstens einem der vorhergehenden Ansprüche, dadurch gekennzeichnet, daß das cellulasefreie Xanthan dadurch erhältlich ist, daß man

a) eine Xanthan enthaltene Lösung auf einen pH-Wert außerhalb des Bereiches von 5 bis 9, einstellt

b) anschließend eine Temperaturbehandlung bei erhöhter Temperatur durchführt

c) gegebenenfalls abkühlt

d) gegebenenfalls neutralisiert und gegebenenfalls

e) das Xanthan in ein turbulentes Fällbad einträgt.

5. Verfahren nach wenigstens einem der vorhergehenden Ansprüche, dadurch gekennzeichnet, daß der aufgesprühte Hilfsstoff Wasser ist.

6. Verfahren nach wenigstens einem der vorhergehenden Ansprüche, dadurch gekennzeichnet, daß als Hilfsstoff ein Komplexbildner eingesetzt wird.

7. Verfahren nach wenigstens einem der vorhergehenden Ansprüche, dadurch gekennzeichnet, daß als Hilfsstoff ein Phosphat eingesetzt wird.

8. Verfahren nach wenigstens einem der vorhergehenden Ansprüche, dadurch gekennzeichnet, daß als Hilfsstoff ein Hydrogen- oder Dihydrogenphosphat eingesetzt wird.

9. Verfahren nach wenigstens einem der vorhergehenden Ansprüche, dadurch gekennzeichnet, daß als Hilfsstoff eine Carbonsäure mit mehr als einer funktionellen Gruppe in Kombination mit einem Alkalicarbonat oder Alkalihydrogencarbonat eingesetzt wird.

10. Verfahren nach wenigstens einem der vorhergehenden Ansprüche, dadurch gekennzeichnet, daß die im Produkt verbleibende Menge des Hilfsstoffs, bezogen auf die Mischung von Xanthan und Cellulosederivat, 0 - 20 Gew.-% beträgt.

11. Verfahren nach wenigstens einem der vorhergehenden Ansprüche, dadurch gekennzeichnet, daß die Mischung cellulasefreies Xanthan und wenigstens ein Cellulosederivat in einem Verhältnis von 1:4 bis 4:1, vorzugsweise 1:2 bis 2:1, beträgt.

12. Verwendung einer gemäß Anspruch 1 hergestellten Mischung zur Viskositätseinstellung in Lebensmitteln, Kosmetika oder Pharmazeutika.

13. Mischung aus Xanthan und einem Cellulosederivat, dadurch gekennzeichnet, daß sie eine Hysteresefläche mit negativem Vorzeichen aufweist bei Messung der Schubspannung in Abhängigkeit von der Schergeschwindigkeit, wobei bei 20°C an einer 1-gew.-%igen wäßrigen Lösung die Schubspannung in Abhängigkeit von der Schergeschwindigkeit mittels eines Rotationsviskosimeters mit Couette-System nach DIN 53 018 kontinuierlich innerhalb von 3 Minuten im Schergeschwindigkeitsbereich von $\gamma$ = 0 bis 50 s$^{-1}$ (Aufwärtskurve) und unmittelbar anschließend innerhalb von 3 Minuten von 50 bis 0 s$^{-1}$ (Abwärtskurve) gemessen wird.

FIG.1

Gesamtkonzentration (%)

Hysteresefläche H (mPa/s)

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | EP-A-0 048 616 (MERCK)<br>* Zusammenfassung *<br>--- | 1,7,12 | C 08 L 5/00<br>C 08 L 1/28<br>C 08 K 3/32<br>C 08 K 5/09<br>C 08 K 5/17<br>A 23 L 1/054<br>A 61 K 9/00 |
| Y | EP-A-0 254 603 (RHONE-POULENC)<br>* Seite 3, Zeilen 39-41,59-63 *<br>--- | 1,7,12 | |
| A | US-A-2 807 591 (J.E. HENRY)<br>* Spalte 2, Zeilen 14-29; Spalte 3, Zeilen 22-49; Spalte 4, Zeilen 24-36 *<br>--- | | |
| A | FR-A-2 299 366 (RHONE-POULENC)<br>* Ansprüche 1,2 *<br>--- | | |
| A | FR-A-2 442 955 (C.E.C.A.)<br>* Seite 3, Zeilen 12-22 *<br>------ | | |

DOMAINES TECHNIQUES RECHERCHES (Int. Cl.5)

C 08 L
C 08 K

Le présent rapport a été établi pour toutes les revendications

| Lieu de la recherche | Date d'achèvement de la recherche | Examinateur |
|---|---|---|
| LA HAYE | 01-06-1990 | SOMERVILLE F.M. |